# EUROPEAN PATENT SPECIFICATION

(11) **EP 1 954 830 B1**
(45) Date of publication and mention of the grant of the patent: **26.10.2011**
(21) Application number: 06829113.7
(22) Date of filing: 23.11.2006
(51) Int. Cl.: C12Q 1/68, G01N 33/574, G01N 33/68

(54) **METHODS AND KITS FOR BREAST CANCER PROGNOSIS**
VERFAHREN UND KITS ZUR BRUSTKREBSPROGNOSE
PROCEDES ET TROUSSES POUR LE PRONOSTIC DU CANCER DU SEIN

(30) Priority: 03.12.2005 EP 05026423
(43) Date of publication of application: 13.08.2008
(73) Proprietor: Sividon Diagnostics GmbH, 50829 Köln (DE)
(72) Inventor: GEHRMANN, Mathias, 51373 Leverkusen (DE); VON TOERNE, Christian, 42659 Solingen (DE)
(74) Representative: von Kreisler Selting Werner
(86) International application number: PCT/EP2006/011230
(87) International publication number: WO 2007/062777

(56) References cited:
- DISIS M L ET AL: "High-titer HER-2/neu protein-specific antibody can be detected in patients with early-stage breast cancer." JOURNAL OF CLINICAL ONCOLOGY : OFFICIAL JOURNAL OF THE AMERICAN SOCIETY OF CLINICAL ONCOLOGY NOV 1997, vol. 15, no. 11, November 1997 (1997-11), pages 3363-3367, XP008075953 ISSN: 0732-183X
- MENARD S ET AL: "ROLE OF HER2 GENE OVEREXPRESSION IN BREAST CARCINOMA" JOURNAL OF CELLULAR PHYSIOLOGY, LISS, NEW YORK, NY, US, vol. 182, no. 2, February 2000 (2000-02), pages 150-162, XP009012973 ISSN: 0021-9541
- PEROU C M ET AL: "Molecular portraits of human breast tumours" NATURE, NATURE PUBLISHING GROUP, LONDON, GB, vol. 406, no. 6797, 17 August 2000 (2000-08-17), pages 747-752, XP002235791 ISSN: 0028-0836
- WANG ET AL: "Gene-expression profiles to predict distant metastasis of lymph-node-negative primary breast cancer" LANCET THE, LANCET LIMITED. LONDON, GB, vol. 365, no. 9460, 19 February 2005 (2005-02-19), pages 671-679, XP005081323 ISSN: 0140-6736 cited in the application
- DATABASE MEDLINE [Online] US NATIONAL LIBRARY OF MEDICINE (NLM), BETHESDA, MD, US 31 January 2002 VAN 'T VEER LAURA J ET AL: 'Gene expression profiling predicts clinical outcome of breast cancer.' Database accession no. NLM11823860 & NATURE 31 JAN 2002, vol. 415, no. 6871, 31 January 2002 (2002-01-31), pages 530-536, ISSN: 0028-0836
- DATABASE MEDLINE [Online] US NATIONAL LIBRARY OF MEDICINE (NLM), BETHESDA, MD, US 1986 ITO T ET AL: 'Class distribution of immunoglobulin-containing plasma cells in the stroma of medullary carcinoma of breast.' Database accession no. NLM3013350 & BREAST CANCER RESEARCH AND TREATMENT 1986, vol. 7, no. 2, 1986, pages 97-103, ISSN: 0167-6806

## Description

### Technical Field of the Invention

The present invention relates to the field of prognosis of a proliferative disease in a patient. More specifically, the present invention relates to methods for the identification of the likely outcome of breast cancer in a breast cancer patient. The invention also relates to methods for the identification of the likely outcome of breast cancer in a patient on the basis of the ERBB2 status of the patient and expression levels of immune genes in tumour tissue of said patient.

### Background of the Invention

Breast cancer is one of the leading causes of cancer death in women in western countries. More specifically, breast cancer claims the lives of approximately 40,000 women and is diagnosed in approximately 200,000 women annually in the United States alone. Over the last few decades, adjuvant systemic therapy has led to markedly improved survival in early breast cancer [2, 3]. This clinical experience has led to consensus recommendations offering adjuvant systemic therapy for the vast majority of breast cancer patients [1]. In breast cancer, a multitude of treatment options are available which can be applied in addition to the routinely performed surgical removal of the tumour and subsequent radiation of the tumour bed. Three main and conceptually different strategies are endocrine treatment, chemotherapy and treatment with targeted therapies. Prerequisite for treatment with endocrine agents is expression of hormone receptors in the tumour tissue i.e. either estrogen, progesterone, or both. Several endocrine agents with different mode of action and differences in disease outcome when tested in large patient cohorts are available. Tamoxifen is one of the oldest endocrine drugs that significantly reduced the risk of tumour recurrence. Apparently, even more effective are aromatase inhibitors which belong to a new endocrine drug class. In contrast to Tamoxifen which is a competitive inhibitor of estrogen binding, aromatase inhibitors block the production of estrogen itself, thereby reducing the growth stimulus for estrogen receptor positive tumour cells. Recent clinical trials have demonstrated an even better disease outcome for patients treated with these agents compared to patients treated with Tamoxifen. Still, some patients experience a relapse despite endocrine treatment and in particular, these patients might benefit from additional therapeutic drugs. Chemotherapy with anthracyclines, taxanes and other agents have been shown to be efficient in reducing disease recurrence in estrogen receptor positive as well as estrogen receptor negative patients. The NSABP-20 study compared Tamoxifen alone against Tamoxifen plus chemotherapy in node negative estrogen receptor positive patients and showed that the combined treatment was more effective than Tamoxifen alone.

A new approach is the treatment of breast cancer patients with monoclonal antibodies which are directed against tumour associated cell surface molecules or against cell surface molecules on endothelial cells of the tumour vasculature.

Recently, the systemically administered monoclonal antibody Trastuzumab (Herceptin; Genentech, South San Francisco, CA, USA) directed against the HER2 antigen on the surface of tumour cells have been shown to reduce the risk of recurrence in patients with HER2 over expressing tumours. At the 2005 American Society of Clinical Oncology meeting, two reports described a 50% reduction in risk of relapse during Trastuzumab therapy in trials comprising around 8000 breast cancer patients. The HER2 antigen is encoded by the ERBB2 gene which is located on chromosome 17q12-q21. Several studies performed in various laboratories have demonstrated that 25 to 30% of all breast and ovarian malignancies show the amplification and over expression of this gene [4, 5]. Amplification of the ERBB2 gene is found in more than 90% of cases that have ERBB2 protein over expression [6, 7], but in normal breast the expression of ERBB2 is due to a transcriptional activation [8]. ERBB2 over expression in women with both node-positive [4, 5] and node-negative [9] breast cancer is associated with a poor prognosis, and several studies have found a correlation between ERBB2 over expression and a shorter disease-free period and shorter overall survival [10, 11].

Various methods are available in the art to evaluate ERBB2 status [12], although immunohistochemistry (IHC; for protein over expression) and fluorescence *in situ* hybridisation (FISH; for gene amplification) are most commonly used. Reports of false-positive Herceptest cases led to suggestions that Herceptests yielding a 2+ score should also be studied by FISH [13]. Tubbs and colleagues [14], called for the US Food and Drug Administration (FDA) to mandate the retraction of the earlier accepted criteria for Trastuzumab therapy, namely that of Herceptests yielding a 2+ score, unless those cases were also confirmed by FISH.

The FDA-approved FISH assay, PathVysion (Vysis, Inc., Downers Grove, IL, USA), is a dual-probe system for the simultaneous enumeration of the ERBB2 gene and the centromeric region of chromosome 17, defining ERBB2 amplification as a ratio of ERBB2 gene copies per chromosome 17 centromere [15]. Another FDA-approved FISH assay, INFORM (Ventana Medical Systems, Tucson, AZ, USA), defines ERBB2 amplification as a mean absolute ERBB2 gene copy number of more than four spots per nucleus, without centromere 17 correction. FISH with the PathVysion probe and immunohistochemical assay with Herceptest are highly concordant for cases showing 3+ IHC scores and for negative cases, although the 2+ IHC score group includes both ERBB2 amplified and non-amplified tumours, showing a relatively high rate of discordance [16, 17]. The mechanisms for ERBB2 expression in non-amplified tumours scored 2+ by IHC are unclear and may involve increased gene dosage by chromosome 17 polysomy [18].

The administration of Trastuzumab may be considered as a kind of passive immunotherapy. Active immunotherapy, e.g. vaccination approaches have not yet been developed successfully due in part to a limited understanding of the functional impact of the immune system on breast cancer. Histological and flow cytometry studies have documented that human breast cancers are infiltrated by a heterogeneous population of immune cells, consisting of different proportions of T cells, B cells, natural killer (NK) cells, and macrophages. Apparently, the immune system is capable of responding to breast cancer as evidenced by systemic, regional, and intra-tumoural leukocyte activation and the presence of leukocytes infiltrating breast tumours has been associated with a favourable prognosis in some studies. However, the prognostic significance of lymphoid infiltrates at the tumour site and the relative importance of these infiltrates as an indicator of host immune status remain controversial [14, 15, 16, 17]. Up to now, for individual patients, there s no predictable relationship between leukocyte composition and prognosis of the disease.

Since Trastuzumab therapy is expensive, the provision of this drug to all ERBB2 over expressing breast cancer patients would result in an enormous economic burden for the health care system. Consequently, a diagnostic test capable to distinguish between ERBB2 over expressing breast tumours with either good or bad prognosis could help to prioritise the urgency of Trastuzumab treatment.

Slamon et al. [9] showed that ERBB2 gene amplification is associated with an increased risk of relapse and death for patients with early-stage breast cancer. It was, however, not disclosed that the expression level of antibodies can be used as a prognostic marker in ERBB2 positive (ERBB2(+)) cancer patients.

WO05/001138 describes the identification of gene expression patterns which may be used to predict survival of subjects afflicted with breast cancer and the likelihood of breast cancer recurrence, in particular in the estrogen receptor (ER) positive subgroup of patients. Methods disclosed in WO05/001138 use the expression level of marker genes for the prognosis of breast cancer only in the ER-positive sub-group of patients. An alternative categorization of patients into a "ERBB2 positive" and "ERBB2 negative" group, however, is not disclosed or suggested.

Disis et al. [18] found that HER-2/neu protein over expression is associated with aggressive disease and is an independent predictor of poor prognosis in several subsets of patients. It is, however, not disclosed that the level of antibodies present in tumour tissue can be used as an indicator for disease outcome in a subset of cancer patients having ERBB2(+) tumours.

Aaltomaa et al. [15] investigated the prognostic value of lymphocyte infiltrates (LI) for recurrence-free survival (RFS) and breast cancer related survival in breast cancer patients. It was shown that the prognostic value of LI could be significantly improved if the patients were categorized into groups of patients having "fast" and "slow proliferating" tumours, respectively. Both, the determination of the proliferation velocity and the determination of the extent of lymphocyte infiltration are difficult experimental procedures, cumbersome to perform and they require significant experience of the laboratory personnel.

Hence, there is a need in the art to provide methods for the identification of a likely outcome of breast cancer in a patient which are reliable and easy to perform experimentally.

### Summary of the Invention

The present invention is based on the unexpected finding that classification of ERBB2(+) breast cancer tissue samples into "good" and "bad outcome" groups can be achieved by measuring the expression level of immunoglobulin genes or their protein products in said breast cancer tissue samples, e.g. antibody heavy or light chain genes, preferentially of the subtype IgM or IgG. In methods of the invention ERBB2 status is determined by methods known in the art, such as immunohistochemistry (IHC), fluorescence *in situ* hybridisation (FISH), chromogenic *in situ* hybridisation (CISH), mRNA expression analysis or any other appropriate method. Similar experimental techniques can be applied for the determination of the relative abundance of immunoglobulin mRNA transcripts or their protein products in the breast cancer tissue. If the tumour is classified as ERBB2(+), the patient prognosis is subsequently deduced from the relative abundance of at least one antibody or immunoglobulin in the breast cancer tissue or sample.

The present invention thus relates to method according to claim 1 for the identification of a likely outcome of breast cancer in a breast cancer patient comprising the steps of (a) determining the ERBB2 status of a tumour of said patient, and (b) determining in a breast cancer tissue sample of said patient the expression level of at least one immunoglobulin in said tumour of said patient at least if said ERBB2 status of said tumour is positive, wherein said identification of a likely outcome is based on said expression level if the ERBB2 status of said tumour is positive. If the tumour is determined to be ERBB2(-), no identification of a likely outcome will be deduced from the expression level of said immunoglobulin in said tumour.

Use of kits and apparatus for performing the above methods are further aspects of the invention.

### Brief description of the figures

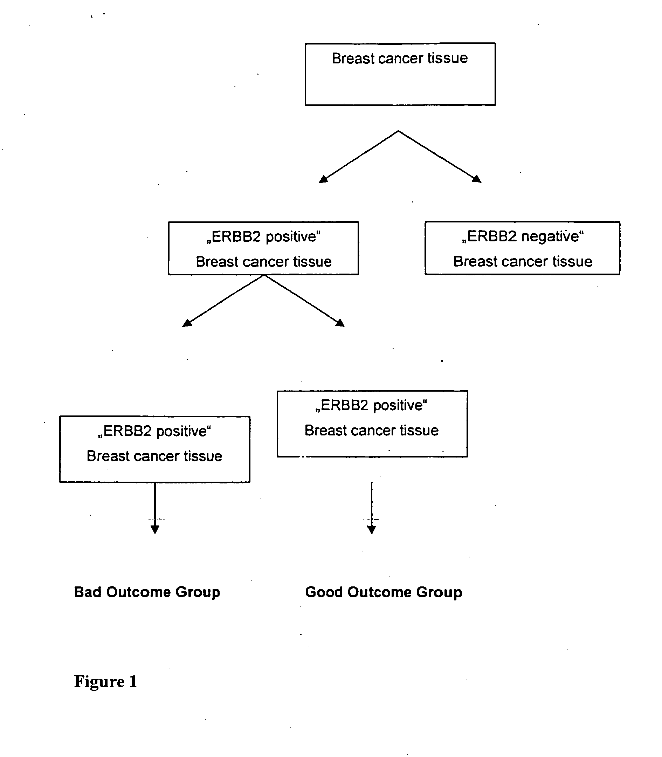

Figure 1: Classification scheme for prognosis of breast cancer tissues according to methods of the invention.
Figure 2: Classification of breast cancer tissues into an ERBB2 positive or negative class based on RNA expression measurements in Cohort I.
Figure 3: ROC curve for determination of immunoglobulin RNA cut-off in Cohort I.
Figure 4: Kaplan Meier survival curve for ERBB2 positive breast cancer patients classified into a "good" or "bad outcome" group in Cohort I.
Figure 5: Classification of breast cancer tissues into a ERBB2 positive or negative class based on RNA expression measurements in Cohort II.
Figure 6: Receiver Operator Characteristic (ROC) curve for determination of immunoglobulin RNA cut-off in Cohort II.
Figure 7: Odds ratio for 60 months disease free survival (DFS) for ERBB2 positive portion of Cohort II.

### Detailed Description of the Invention

"Breast cancer tissue", within the meaning of the invention, relates to tissue obtained from the human body by resection or biopsy which contains breast cancer cells. The tissue may originate from a carcinoma *in situ*, an invasive primary tumour, a recurrent tumour, lymph nodes infiltrated by tumour cells, or a metastatic lesion. The meaning of "breast cancer tissue" is independent of the "ERBB2 status", within the meaning of the invention, relates to the classification of breast cancer tissue to an ERBB2 positive (ERBB2(+)) or ERBB2 negative (ERBB2(-)) class based on the determination of the expression level of the ERBB2 gene as measured by methods such as immunohistochemistry (IHC), fluorescence *in situ* hybridisation (FISH), chromosome *in situ* hybridisation (CISH), gene chip or other methods known to the person skilled in the art. Such methods relate to the determination of ERBB2 protein, DNA or mRNA from breast cancer tissue. Immunohistochemical classification may be done by the use of the HercepTest™ (DakoCytomation, Glostrup, Denmark). Reading of the results of the immunohistochemical reaction of HER2 can be done using a grading system in which the degree of gene amplification corresponds to the staining reaction of the HercepTest™. As recommended by the manufacturer of the test, staining should only be at the membrane and is assessed on a scale of 0 to 3+, dependent on the intensity of staining in more than 10% of invasive tumour cells. Any cytoplasmic staining is ignored. 3+ is positive (strong), 2+ is positive (weak) and 1+ as well as 0 is negative. The HercepTest™ is commercially available and FDA approved for prediction of Trastuzumab sensitivity, as is the Pathway™ (Ventana Medical Systems, Tucson, AZ) and InSite™ test (Biogenex, San Ramon, CA, USA). As an alternative to immunohistochemical detection of HER2, a FISH test can be used to determine at the gene level if the ERBB2 is amplified and the ERBB2 status is correspondingly negative or positive. Commercially available FISH tests include the PathVysion™ HER2 FISH test (Abbott-Vysis, Downers Grove, IL, USA), the INFORM^{®} HER2 FISH (Ventana Medical Systems) and the PharmDX™ HER2 FISH test (DakoCytomation). Another possibility for the determination of the ERBB2 status is the use of PCR, in particular real time PCR with primers and probe specific for ERBB2 DNA, or alternatively specific for ERBB2 encoding mRNA.

"Immunoglobulin", within the meaning of the invention, relates to polypeptides or mRNA transcripts encoded by immunoglobulin genes. Immunoglobulins can also be parts or fragments of said polypeptides or mRNA transcripts. Immunoglobulin genes encode the antibody molecules which are the antigen-recognition molecules produced by B cells. RNA transcripts from an immunoglobulin gene may be molecules coding for heavy or light chains of antibodies. Light chain transcripts may encode the kappa or lambda isoform of an antibody, heavy chain transcripts may encode the µ- or γ-isoform of an antibody. Particularly suited regions for the detection and quantification of immunoglobulin transcripts are nucleic acid sequences coding for the constant domains of heavy or light chains respectively. A non-exclusive list of exemplary immunoglobulin transcript sequences is listed in Table 1. The relative abundance of immunoglobulins in breast cancer tissue may also be determined by measuring the abundance of peptides encoded by immunoglobulin genes. Immunohistochemistry may be applied for this purpose, utilizing antibody molecules specific for immunoglobulin gene transcripts as detection tool. Particularly suited antibody molecules are specific for the constant regions of immunoglobulin heavy or light chains.

"Relative abundance", within the meaning of the invention, defines the relationship of the total expression of a particular immunoglobulin in a tumour tissue sample in comparison with a given threshold value that is specific to the selected immunoglobulin and overall method, but not to a specific tumour tissue sample. An expression that is higher than the given threshold value will be denoted as "high expression" whereas an expression that is lower than the given threshold will be denoted as "low expression."

"Determination of an expression level" of a gene in a tissue, within the meaning of the invention shall be understood to be any determination of the amount of mRNA coding for said gene, or a part of said gene, in said tissue; or any determination of the amount of the protein product coded for by said gene in said tissue. Various methods to determine the expression level of a gene in a tissue are known in the art. These methods comprise, without limitation, PCR methods, real-time PCR methods, gene chip experiments, immunohistochemistry (IHC), fluorescence *in situ* hybridisation (FISH), chromosome *in situ* hybridisation (CISH) and other methods known to the person skilled in the art.

A "gene chip", or "microarray" within the meaning of the invention, shall be understood as being any type of solid support material, comprising a multitude of local features, each feature comprising immobilized nucleic acid probes. These nucleic acid probes are able to bind to free nucleic acids in a sample, wherein such binding can be detected by suitable methods. Various technical implementations of gene chips are known to the person skilled in the art and commercially available. One well known example of a gene chip is the GeneChip^{™} of Affymetrix, Inc. (Santa Clara, CA). Microarray or gene chip technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labelled probe used to detect a cDNA sequence from the sample that hybridises to a nucleic acid sequence at a known location on the microarray. Typically, the intensity of the signal is proportional to the quantity of cDNA, and thus mRNA, expressed in the sample cells. A large number of such techniques are known to the person skilled in the art and useful within the context of the present invention.

"Classification", within the meaning of the invention, denotes the mapping of an unknown sample to one of at least two pre-defined groups. In the context of the invention, classification is carried out on basis of the expression level of certain immunoglobulins or on the basis of the expression level of the ERBB2 gene.

"Good outcome" and "bad outcome", within the meaning of the invention, relates to the classify-cation of breast cancer patients in at least two different groups according to the probability that the breast cancer will have a favourable or unfavourable outcome. Patients in a "good outcome" group will usually have, e.g. better overall survival, lower chance of the occurrence of metastases within 60 months, etc..

The present disclosure relates to methods for the identification of a likely outcome of breast cancer in a breast cancer patient comprising the steps of
(a) determining the ERBB2 status of a tumour of said patient, and
(b) determining the expression level of at least one immunoglobulin in said tumour of said patient at least if said ERBB2 status of said tumour is positive,
   wherein said identification of a likely outcome is based on said expression level if the ERBB2 status of said tumour is positive. It is to be understood that the denomination of the steps of the method as (a) and (b) shall not indicate that these steps have to be performed in a certain sequence. Quite contrary, step (a) and (b) can be performed in any timely order or, preferably, can be performed at the same time, e.g. in parallel, such as on a gene chip.
, said identification of a likely outcome is based on said expression level only if the ERBB2 status is positive. If the ERBB2 status in the tumour is determined to be negative, the expression level of the immunoglobulin may be disregarded and/or other methods for the identification of a likely outcome may be employed.

According to another embodiment of the invention, the step of determining the ERBB2 status of said tumour is prior to the step of determining the expression level of at least one immunoglobulin.

In preferred embodiments of the invention in step (b) the expression level of more than one immunoglobulin is determined. This may improve the statistical quality and the reliability of the identification method.

In methods of the disclosure, the identification of a likely outcome preferably relates to
(a) a determination of the likelihood of recurrence of a tumour in said patient, and/or
(b) the prediction of long term survival of said patient, and/or
(c) the identification of a likely outcome of breast cancer in a breast cancer patient after surgery, and/or
(d) the prognosis of breast cancer.

In methods of the invention, the identification of the likely outcome comprises a classification of the patient into one of at least two groups, said two groups corresponding to a "good outcome" group and a "bad outcome" group, respectively. A "good outcome" group may also be seen as a group having "low risk" of recurrence of cancer and a "bad outcome" group may likewise be regarded as being a group having "high risk" of recurrence of cancer. These expressions may be used interchangeably.

In methods of the invention, the determination of the ERBB2 status may comprise a comparison of an ERBB2 expression level with a predetermined threshold value. Tumours having measured ERBB2 expression levels above said threshold level may be categorized as being "ERBB2 positive" and tumours having measured ERBB2 expression levels equal to or below said threshold level may be categorized as being "ERBB2 negative". The threshold level is preferably predetermined, i.e. a fixed value is given to said threshold value prior to the evaluation of the ERBB2 expression level. Said predetermined value can be obtained from previous experiments or from literature.

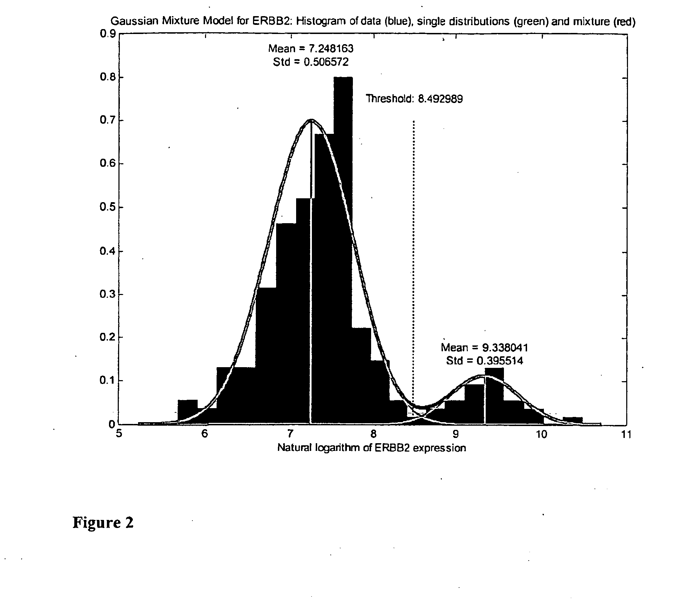

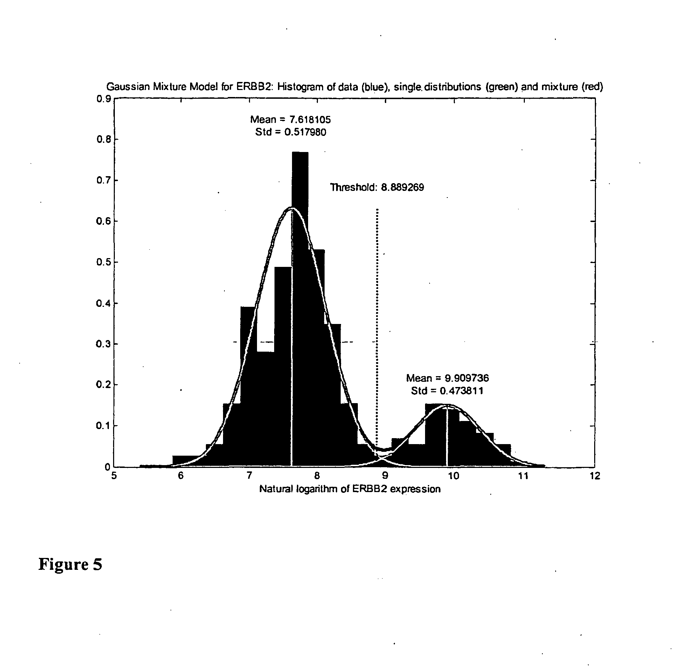

In preferred methods of the invention, said threshold value is determined from a Gaussian mixture model of a histogram determined from a suitable cohort of breast cancer patients, wherein the histogram comprises the ERBB2 expression levels in tumours of said cohort. An example of how this may be achieved is illustrated in Figs. 2 and 5. A histogram is constructed using the expression levels of ERBB2 in tumours of breast cancer patients in a representative cohort of patients. The relative number of patients in "bins" of the histogram is plotted over the natural logarithm of the ERBB2 expression level. A Gaussian Mixture Model (GMM) was used to separate the two modes of the bimodal distribution. Parameters for the distinct distributions and the threshold value were estimated with an Expectation maximization (EM) algorithm, which is well known to the person skilled in the art. The threshold value may alternatively be obtained using other methods known to the skilled artisan.

In preferred embodiments of the invention, said determination of said expression level of at least one immunoglobulin comprises the determination of the amount of mRNA coding for said immunoglobulin. Any suitable method for the determination of an expression level known to the person skilled in the art may be employed.

In other preferred embodiments of the invention, said determination of said expression level of at said least one immunoglobulin comprises the determination of the amount of mRNA coding for at least a part of said immunoglobulin.

In further preferred embodiments of the invention the ERBB2 status is determined by polymerase chain reaction (PCR), or by real time PCR, or by immunohistochemistry, or by fluorescence *in situ* hybridisation (FISH), or by chromosome *in situ* hybridisation (CISH), or on a gene chip. If desired several of the methods above can be employed for the determination.

In other preferred embodiments of the invention, the expression level of said immunoglobulin is determined by PCR or real time PCR or by immunohistochemistry, or on a gene chip.

Particularly preferred are methods in which the ERBB2 status *and* the expression level of said immunoglobulin are determined at the same time, e.g. on the same gene chip. This helps to reduce labour and costs.

In a preferred embodiment of the invention, a cancer is classified to be of the "bad outcome" group if the expression level of said at least one immunoglobulin is below a predetermined threshold level.

In other preferred methods, a cancer is classified to be of the "bad outcome" group if the combined value of the expression levels (or the average expression level) of at least two immunoglobulin genes is below a predetermined threshold level.

In preferred methods of the invention the immunoglobulin is an antibody.

In further preferred embodiments of the invention, said immunoglobulin is selected from the group consisting of IGH@, IGHG1, IGHG4, IGHM, IGHD, IGHG3, IGJ, IGKC, IGLC2, IGLV@, IGLJ3, IGSF2, IGSF3, IGSF4B and IGSF4C, more preferably from the group comprising IGCK, IGHG1 and IGH@.

Another aspect of the invention relates to the use of a kit comprising
(a) means for the determination of the ERBB2 status of a breast cancer patient from a tumour sample of said patient,
(b) means for the determination of the expression level of at least one immunoglobulin in a tumour sample of said patient, and
(c) printed material or a data carrier comprising instructions for performing a method according to any of claims 1-13.

Such kits can be used to perform the above described methods of the invention. Preferably, said means for the determination of the EBB2 status and said means for the determination of the expression level of at least one immunoglobulin are the same. Such means can comprise, e.g., a gene chip.

Another aspect of the invention relates to the use of an apparatus for the identification of a likely outcome of breast cancer in a breast cancer patient, said device comprising
(a) means for the determination of the ERBB2 status of a breast cancer patient from a tumour sample of said patient,
(b) means for the determination of the expression level of at least one immunoglobulin in a tumour sample of said patient, and
(c) computing means capable of identifying said likely outcome, wherein said identification is based on the expression level of said at least one immunoglobulin if said ERBB2 status is positive.

The computing means are preferably connected to the means for the determination of the ERBB2 status and to the means for the determination of the expression level of at least one immunoglobulin by singal transmitting means, such that the result to the respective determinations is readily available to the computing means for the identification of said likely outcome of said breast cancer.

In preferred methods of the invention, the determination of the ERBB2 status of the tumour and the determination of the expression level of the at least one immunoglobulin is performed on samples from said patients. These samples are preferably previously available.

The determination of the ERBB2 status of the tumour and the determination of the expression level of the at least one immunoglobulin is preferably *in vitro.*

The identification of a likely outcome of the breast cancer is determined separately from diagnosing the disease, e.g. after breast cancer has been diagnosed in said patient. In preferred embodiments of the invention, the identification does not involve diagnosis.

The invention is further illustrated by way of the following examples.

### Examples

### Example 1: Prognosis of ERBB2 positive breast Cancer - Cohort I

### Patient selection and RNA isolation from frozen tumour tissue sections and gene expression measurement utilizing HG-U133A microarrays of Affymetrix

Frozen sections were taken for histology and the presence of breast cancer was confirmed in samples from 205 patients with breast cancer. Tumour cell content exceeded 30 % in all cases and was above 50 % in most cases. Approximately 50 mg of snap frozen breast tumour tissue was crushed in liquid nitrogen. RLT-Buffer (QIAGEN, Hilden, Germany) was added and the homogenate spun through a QIAshredder column (QIAGEN, Hilden, Germany). From the eluate total RNA was isolated by the RNeasy Kit (QIAGEN, Hilden, Germany) according to the manufacturers instruction. RNA yield was determined by UV absorbance and RNA quality was assessed by analysis of ribosomal RNA band integrity on the Agilent Bioanalyzer (Palo Alto, CA, USA).

Starting from 5 µg total RNA labelled cRNA was prepared for all 205 tumour samples using the Roche Microarray cDNA Synthesis, Microarray RNA Target Synthesis (T7) and Microarray Target Purification Kit according to the manufacturer's instruction. In brief, synthesis of first strand cDNA was done by a T7-linked oligo-dT primer, followed by second strand synthesis. Double-stranded cDNA product was purified and then used as template for an in vitro transcription reaction (IVT) in the presence of biotinylated UTP. Labelled cRNA was hybridised to HG-U133A arrays (Santa Clara, CA, USA) at 45°C for 16 h in a hybridisation oven at a constant rotation (60 r.p.m.) and then washed and stained with a streptavidin-phycoerythrin conjugate using the GeneChip fluidic station. The arrays were scanned at 560 nm using the GeneArray Scanner G2500A from Hewlett Packard. The readings from the quantitative scanning were analysed using the Microarray Analysis Suit 5.0 (MAS 5.0) from Affymetrix. In the analysis settings the global scaling procedure was chosen which multiplied the output signal intensities of each array to a mean target intensity of 500. Array images were visually inspected for defects and quality controlled using the Refiner Software from GeneData. Routinely we obtained over 50 percent present calls per chip as calculated by MAS 5.0.

### Determination of the ERBB2 status based on mRNA measurement

Of the 205 samples in this cohort, measurements of gene expression levels were taken. As in the preceding example, a Gaussian mixture model was used on the (natural) logarithmised absolute expression of ERBB2 (Affymetrix probeset ID 216836_s_at) to separate the two modes of the distribution. From the 205 expression values, a threshold value of 8.49 on a (natural) log scale (about 4880 on a linear scale) was found to be the likeliest.

Samples that showed an expression equal to or above that value were labelled "ERBB2 positive", the remaining samples were labelled "ERBB2 negative". Of the 205 samples, 24 samples were ERBB2(+), those 191 samples that had been labelled ERBB2 negative were not further investigated.

### Classification of ERBB2 positive Breast Cancer samples into "good outcome" and "bad outcome" groups

For the ERBB2(+) samples, expression profiles of genes related to the immune system were measured by the HG_U133A GeneChip^{™}. The genes measured are listed in Table 1, together with a unique identifier for the respective probe set on the Affymetrix GeneChip^{™}.

**Table 1:**

| Probeset ID | Gene Symbol | Gene Name(s) |
|---|---|---|
| 214973_x_at | IGHD | Immunoglobulin heavy constant delta |
| 216892_at | IGH@ | Immunoglobulin heavy locus |
| 216541_x_at | IGHG1 | immunoglobulin heavy constant gamma 1 (Glm marker) |
| 212592_at | IGJ | Immunoglobulin J polypeptide, linker protein for immunoglobulin alpha and mu polypeptides |
| 215621_s_at | IGHD | immunoglobulin heavy constant delta |
| 211648_at | IGHM | Immunoglobulin heavy constant mu /// Immunoglobulin heavy constant mu |
| 217198_x_at | IGH@ /// | immunoglobulin heavy locus /// immunoglobulin heavy constant gamma 1 (Glm marker) |
| | IGHG1 | |
| 217442_at | IGSF4B | Immunoglobulin superfamily, member 4B |
| 214677_x_at | IGLC2 | immunoglobulin lambda constant 2 (Kern-Oz- marker) |
| 211640_x_at | IGHG1 | immunoglobulin heavy constant gamma 1 (Glm marker) /// immunoglobulin heavy constant gamma 1 (Glm marker) |
| 211634_x_at | IGHM | immunoglobulin heavy constant mu /// immunoglobulin heavy constant mu |
| 209374_s_at | IGHM | immunoglobulin heavy constant mu |
| 215035_at | IGLC2 | Immunoglobulin lambda constant 2 (Kern-Oz- marker) /// Amyloid lambda 6 light chain variable region SAR |
| 221651_x_at | IGKC | immunoglobulin kappa constant |
| 221671_x_at | IGKC | immunoglobulin kappa constant |
| 215121_x_at | IGLC2 | immunoglobulin lambda constant 2 (Kern-Oz- marker) |
| 2H430_s_at | IGHG1 | immunoglobulin heavy constant gamma 1 (Glm marker) |
| 211647_x_at | IGHG1 | immunoglobulin heavy constant gamma 1 (Glm marker) /// immunoglobulin heavy constant gamma 1 (Glm marker) |
| 202421_at | IGSF3 | Immunoglobulin superfamily, member 3 |
| 222293_at | IGSF4C | immunoglobulin superfamily, member 4C |
| 207167_at | IGSF2 | immunoglobulin superfamily, member 2 |
| 217369_at | IGH@ /// | IgM rearranged heavy chain mRNA V-D-J region /// Immunoglobulin heavy locus /// Immunoglobulin heavy constant gamma 1 (Glm marker) /// Immunoglobulin heavy constant gamma 4 (G4m marker) /// Immunoglobulin heavy chain VH3 (H11) /// Rearranged Ig mu-chain (V6-1/Dxp4-DA5/JH6b) /// Similar to immunoglobulin M chain /// Transcribed locus, moderately similar to XP_234649.1 similar to immunoglobulin heavy chain variable region [Rattus norvegicus] /// Immunoglobulin heavy constant mu |
| | IGHG1 /// | |
| | IGHG4 /// | |
| | IGHM | |
| 211146_at | IGKC | Immunoglobulin kappa constant |
| 215721_at | IGHG1 | immunoglobulin heavy constant gamma 1 (Glm marker) |
| 211798_x_at | IGLJ3 | immunoglobulin lambda joining 3 |
| 216846_at | IGLC2 /// | Immunoglobulin lambda light chain V region (Humla203) /// Transcribed locus, weakly similar to XP_213585.2 similar to anti-idiotype immunoglobulin M light chain [Rattus norvegicus] /// Anti-HIV-1 gp120 immunoglobulin E51 lambda light chain /// Immunoglobulin lambda constant 2 (Kern-Oz- marker) /// Immunoglobulin lambda chain mRNA, VJ regions, hybridoma JB32 /// Immunoglobulin lambda variable group /// Hepatitis B surface antigen antibody variable domain /// Isolate TASS immunoglobulin light chain variable region /// Immunoglobulin lambda joining 3 /// Ig lambda chain V-region (VL-HUC) |
| | IGLV@ /// | |
| | IGLJ3 | |
| 211644_x_at | IGKC | HRV Fab 027-VL /// HRV Fab 027-VL /// Similar to CDNA sequence BC012256 /// Similar to CDNA sequence BC012256 /// Anti-rabies virus immunoglobulin rearranged kappa chain V-region /// Anti-rabies virus immunoglobulin rearranged kappa chain V-region /// HRV Fab 026-VL /// HRV Fab 026-VL /// Immunoglobulin kappa constant /// Immunoglobulin kappa constant /// Monoclonal antibody kappa chain variable region /// Monoclonal antibody kappa chain variable region /// Ig kappa chain V-region (VL-COL) /// Ig kappa chain V-region (VL-COL) |
| 211881_x_at | IGLJ3 | immunoglobulin lambda joining 3 |
| 217169_at | IGHG3 | IgM VDJ-region /// Immunoglobulin heavy constant gamma 3 (G3m marker) |
| 216535_at | IGSF4B | immunoglobulin superfamily, member 4B |

Further analysis was based on the expression level of immunoglobulin kappa chain (IGKC; Affymetrix Probeset ID 221671_x_at). Of the 24 ERBB2(+) samples, only 21 could be used because the remaining 4 patients had a follow-up of less than 60 months, so the metastasis information at 60 months of follow-up remained unknown.

For a chosen threshold value for the expression of IGKC, samples that had a lower expression value with respect to this threshold were labelled "Low IGKC", and "High IGKC" for higher expression values. A specificity of at least 90% was assumed (which, in this case, allowed no cases to be wrongfully classified as "good outcome") and the threshold value was chosen that allowed for the greatest sensitivity (under the 90%-specificity constraint).

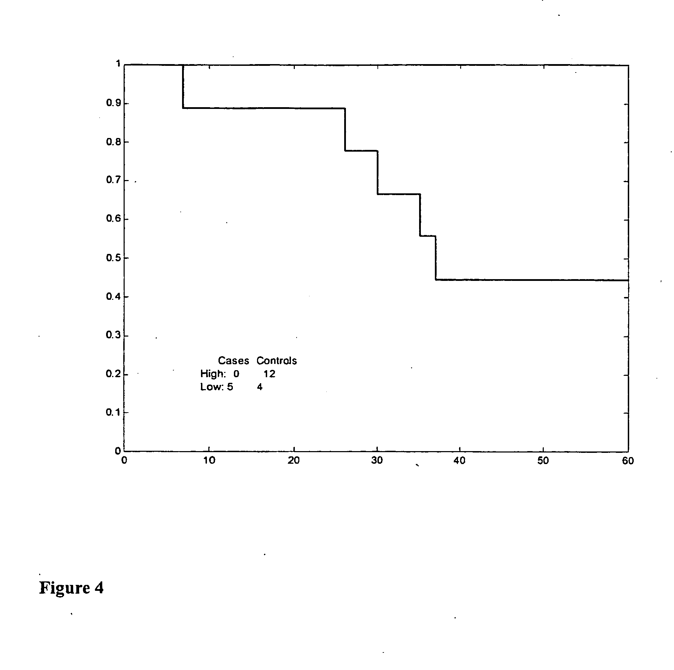

In the case of the 21 ERBB2(+) samples, a threshold value was determined to be 14424, and maximum sensitivity of 75% was obtained (with the "bad outcome" group being the samples with an IGKC expression of less than 14424 and the "good outcome" group being the samples with an IGKC expression of more than 14424). Since no false negatives could were allowed, an odds ratio could not be meaningfully computed because its value would be infinite. Kaplan-Meier curves are given in Figure 4.

The overall prognosis is thus performed as follows: First, measurements of ERBB2 and IGKC are taken. If the ERBB2 expression value is above 4880, the sample is labelled "ERBB2(+)", otherwise it is labelled "ERBB2(-)". If the ERBB2(+) status is positive, the IGKC expression is compared with the threshold value of 14424. Samples showing an expression higher than this value are considered "good outcome", samples showing an expression level equal to or lower than 14424 are considered "bad outcome".

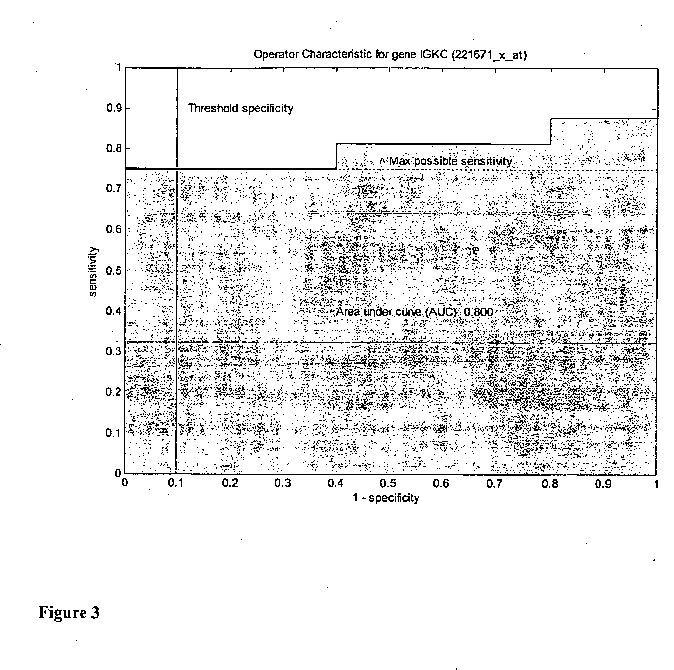

Whilst the above analysis is based on the IGKC gene, other genes coding for immunoglobulins (e.g. genes listed in Table 1) may also be used for the analysis. Table 2 provides the "area under the curve" in Operator Characteristic plots (cf. Figure 3), the maximum sensitivity at 90% specificity and the corresponding cut-off value for expression level measurements for genes listed in Table 1. Also given in Table 2 is the maximum specificity at 90% sensitivity and the corresponding cut-off for the expression level, as determined in this example. Genes providing a sensitivity at 90% specificity of greater than 0.3 are ranked 1 to 6, wherein a low rank indicates a high sensitivity. Genes providing a high sensitivity, i.e. rank 1-6, are preferred. As will be appreciated by the person skilled in the art, more than one of said genes at a time can be used in the analysis.

**Table 2: Cohort I (24 Samples)**

| Probeset ID | Gene Symbol | Rank | Area under Curve (AUC) | Max. Sens. (@ 90% Spec.) | Cut-Off | Max Spec (@ 90% Sens.) | Cut-Off |
|---|---|---|---|---|---|---|---|
| 214973_x_at | IGHD | 1 | 0.820513 | 0.692308 | 312.9 | 0.222222 | 156.7 |
| 216892_at | IGH@ | 2 | 0.820513 | 0.538462 | 81.4 | 0.333333 | 16.6 |
| 216541_x_at | IGHG1 | 4 | 0.820513 | 0.384615 | 92.3 | 0.555556 | 15.3 |
| 212592_at | IGJ | 3 | 0.803419 | 0.461538 | 1254.0 | 0.444444 | 272.4 |
| 215621_s_at | IGHD | 3 | 0.786325 | 0.461538 | 47.3 | 0.444444 | 6.9 |
| 211648_at | IGHM | 3 | 0.709402 | 0.461538 | 28.6 | 0.222222 | 6.1 |
| 217198_x_at | IGH@ /// | 3 | 0.649573 | 0.461538 | 97.8 | 0.222222 | 19.7 |
| | IGHG1 | | | | | | |
| 217442_at | IGSF4B | | 0.632479 | 0.153846 | 27.2 | 0.222222 | 5.2 |
| 214677_x_at | IGLC2 | | 0.726496 | 0.153846 | 24897.0 | 0.333333 | 5089.0 |
| 211640_x_at | IGHG1 | 4 | 0.863248 | 0.384615 | 130.9 | 0.555556 | 16.1 |
| 211634_x_at | IGHM | 4 | 0.820513 | 0.384615 | 432.7 | 0.666667 | 75.2 |
| 209374_s_at | IGHM | 4 | 0.760684 | 0.384615 | 4354.0 | 0.111111 | 299.3 |
| 215035_at | IGLC2 | | 0.683761 | 0 | 65.0 | 0 | 4.1 |
| 221651_x_at | IGKC | | 0.794872 | 0.153846 | 25414.0 | 0.555556 | 10630.0 |
| 221671_x_at | IGKC | | 0.794872 | 0.153846 | 26122.0 | 0.555556 | 10704.0 |
| 215121_x_at | IGLC2 | | 0.760684 | 0.153846 | 32860.0 | 0.444444 | 4391.0 |
| 211430_s_at | IGHG1 | | 0.769231 | 0.230769 | 22246.0 | 0.444444 | 6221.0 |
| 211647_x_at | IGHG1 | 6 | 0.752137 | 0.307692 | 93.2 | 0.111111 | 10.0 |
| 202421_at | IGSF3 | 5 | 0.726496 | 0.333333 | 1009.0 | 0.230769 | 707.4 |
| 222293_at | IGSF4C | 5 | 0.717949 | 0.333333 | 80.0 | 0.153846 | 14.6 |
| 207167_at | IGSF2 | 6 | 0.803419 | 0.307692 | 128.8 | 0.555556 | 49.0 |
| 217369_at | IGH@ /// IGHG1 /// IGHG4 /// IGHM | 6 | 0.803419 | 0.307692 | 474.9 | 0.555556 | 213.5 |
| | | | | | | | |
| | | | | | | | |
| | | | | | | | |
| 211146_at | IGKC | 6 | 0.615385 | 0.307692 | 8.6 | 0.222222 | 4.1 |
| 215721_at | IGHG1 | 6 | 0.521368 | 0.307692 | 21.3 | 0 | 5.1 |
| 211798_x_at | IGLJ3 | | 0.726496 | 0.153846 | 2100.0 | 0.444444 | 190.7 |
| 216846_at | IGLC2 /// | | 0.606838 | 0 | 115.8 | 0 | 9.4 |
| | IGLV@ /// | | | | | | |
| | IGLJ3 | | | | | | |
| 211644_x_at | IGKC | | 0.820513 | 0.153846 | 5594.0 | 0.666667 | 445.8 |
| 211881_x_at | IGLJ3 | | 0.717949 | 0.153846 | 1035.0 | 0.444444 | 188.7 |
| 217169_at | IGHG3 | | 0.649573 | 0.111111 | 57.1 | 0.153846 | 5.0 |
| 216535_at | IGSF4B | | 0.598291 | 0 | 159.4 | 0 | 27.9 |

### Example 2: Prognosis of ERBB2 positive breast Cancer Samples - Cohort II

### Patient selection, RNA isolation from frozen tumour tissue sections, and gene expression measurement utilizing HG-U133A microarrays of Affymetrix

286 frozen breast cancer tissue samples had been selected from the tumour bank at the Erasmus Medical Center (Rotterdam, Netherlands) and had been processed as described by Wang et al. (2005. Gene-expression profiles to predict distant metastasis of lymph-node-negative primary breast cancer, Lancet 365: 671-679). Briefly, total RNA was isolated from 20-40 cryostat sections of 30 µm thickness (50-100 mg) with RNAzol B (Campro Scientific, Veenendaal, Netherlands). Biotinylated targets were prepared by published methods (Affymetrix, Santa Clara, CA, USA) and hybridised to the Affymetrix oligonucleotide microarray U133a GeneChip. Arrays were scanned by standard Affymetrix protocols. Each probe set was treated as a separate gene. Expression values were calculated by use of Affymetrix GeneChip analysis software MAS 5.0. Chips with average intensity of less than 40 or background signal of more than 100 were rejected. For chip normalisation, probe sets were scaled to a target intensity of 600, and scale mask files were not selected.

### Determination of the ERBB2 status based on mRNA measurement

Data of all 286 tissue samples were taken. A Gaussian mixture model for the logarithm of the absolute ERBB2 (Affymetrix probe ID: 216836_s_at) expression was applied to separate the two modes of the bimodal distribution. According to this model, an optimal threshold value for the data set under consideration was an absolute ERBB2 expression of about 7254. Samples that showed an expression level of that particular gene that was at least as high as the chosen threshold were labelled "ERBB2 positive", the remaining samples were consequently labelled "ERBB2 negative". Of the given 286 samples, 51 were found to be in the group ERBB2(+), the remaining 235 samples were in ERBB2(-) and therefore not considered further.

### Classification of ERBB2 positive Breast Cancer samples into "good outcome " or "bad outcome" groups

For the ERBB2(+) samples, expression levels of the genes listed in Table 1 were measured.

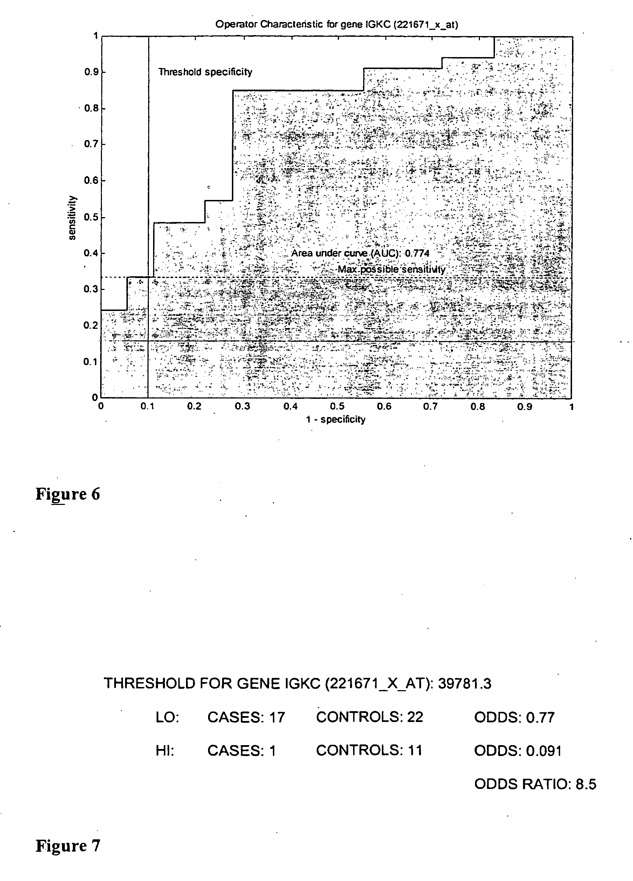

For each of the genes, a classification operator was defined by labelling the genes with respect to their expression relative to a chosen threshold. The optimal threshold for each gene was determined on basis of an operator characteristic derived by choosing different threshold values and computing sensitivity and specificity with respect to the metastasis information for each sample. An example for IGKC (Immunoglobulin kappa chain) is shown in Figure 6.

We allowed a specificity of 90% or above and chose the threshold value that gives the best sensitivity. In the case of IGKC, the best sensitivity was about 30%. Even though this value may seem low, the total case/control statistics given in Figure 7 allows the conclusion that in fact we have an odds ratio of the classifier of 8.5 if we identify LO (i.e. the group that has an expression of IGKC that is below the threshold value of 37485.4) as a "bad outcome group" and consequently HI as a "good outcome" group (containing patients with an expression of IGKC of more than 37485.4).

The overall prognosis would thus proceed as follows: First measurements of ERBB2 and IGKC are taken. If the ERBB2 expression value is above the threshold value of 7254, the sample is labelled "ERBB2(+)", otherwise it is labelled "ERBB2(-)". For the ERBB2 positive samples, the IGKC expression is compared with the threshold of 37485.4. Samples that show an expression higher than this value are labeled "good outcome", samples with an expression level lower than 37485.4 are labeled "bad outcome".

Whilst the above analysis is based on the IGKC gene, other genes coding for immunoglobulins (e.g. genes listed in Table 1) may also be used for the analysis. Table 3 provides the "area under the curve" in Operator Characteristic plots, the maximum sensitivity at 90% specificity and the corresponding cut-off value for expression level measurements for the genes listed in Table 1. Also given in Table 3 are the maximum specificity at 90% sensitivity and the corresponding cut-off for the expression level, as determined in this example. Genes providing a sensitivity at 90% specificity of greater than 0.3 are ranked 1 to 7, wherein a low rank indicates a high sensitivity. Genes providing a high sensitivity, e.g. rank 1-7, are preferred within the context of the invention.

IGCK, IGHG1 and IGH@ provide high sensitivity at 90% specificity in both of examples 1 and 2 (i.e. Cohort I and II). Thus these genes are preferred in methods of the invention.

**Table 3: Cohort II (51 Samples)**

| Probeset ID | Gene Symbol | Rank | Area under Curve (AUC) | Max. Sens. (@ 90% Spec.) | CutOff | Max Spec (@ 90% Sens.) | CutOff |
|---|---|---|---|---|---|---|---|
| 214973_x_at | IGHD | | 0.752525 | 0.242424 | 687.8 | 0.333333 | 94.6 |
| 216892_at | IGH@ | | 0.641414 | 0.181818 | 304.9 | 0.277778 | 13.2 |
| 216541_x_at | IGHG1 | 1 | 0.732323 | 0.484848 | 120.9 | 0.222222 | 15.0 |
| 212592_at | IGJ | | 0.712121 | 0.242424 | 2773.6 | 0.444444 | 371.8 |
| 215621_s_at | IGHD | | 0.60101 | 0.212121 | 74.7 | 0.166667 | 9.3 |
| 211648_at | IGHM | | 0.540404 | 0.166667 | 72.9 | 0.060606 | 7.3 |
| 217198_x_at | IGH@ /// | 6 | 0.624579 | 0.30303 | 170.3 | 0.166667 | 17.5 |
| | IGHG1 | | | | | | |
| 217442_at | IGSF4B | 2 | 0.737374 | 0.454545 | 17.5 | 0.166667 | 9.6 |
| 214677_x_at | IGLC2 | 3 | 0.759259 | 0.393939 | 37438. 0 | 0.444444 | 9551.5 |
| 211640_x_at | IGHG1 | | 0.66835 | 0.212121 | 164.2 | 0.333333 | 13.1 |
| 211634_x_at | IGHM | | 0.646465 | 0.212121 | 487.1 | 0.333333 | 26.1 |
| 209374_s_at | IGHM | | 0.685185 | 0.212121 | 7064.2 | 0.388889 | 618.2 |
| 215035_at | IGLC2 | 4 | 0.671717 | 0.363636 | 23.1 | 0.166667 | 5.6 |
| 221651_x_at | IGKC | 5 | 0.777778 | 0.333333 | 37254. 1 | 0.444444 | 11564.1 |
| 221671_x_at | IGKC | 5 | 0.774411 | 0.333333 | 35208. 7 | 0.444444 | 10851.4 |
| 215121_x_at | IGLC2 | 5 | 0.740741 | 0.333333 | 35894. 3 | 0.388889 | 3257.7 |
| 211430_s_at | IGHG1 | 5 | 0.703704 | 0.333333 | 39837. 2 | 0.111111 | 796.2 |
| 211647_x_at | IGHG1 | 5 | 0.648148 | 0.333333 | 112.8 | 0.055556 | 12.5 |
| 202421_at | IGSF3 | | 0.587542 | 0 222222 | 2285.0 | 0.333333 | 1205.7 |
| 222293_at | IGSF4C | | 0.654882 | 0.151515 | 153.3 | 0.277778 | 24.6 |
| 207167_at | IGSF2 | | 0.550505 | 0.030303 | 195.4 | 0.111111 | 42.4 |
| 217369_at | IGH@ /// | | 0.572391 | 0.121212 | 493.1 | 0.222222 | 142.4 |
| | IGHG1 /// | | | | | | |
| | IGHG4 /// | | | | | | |
| | IGHM | | | | | | |
| 211146_at | IGKC | 7 | 0.680135 | 0.272727 | 15.7 | 0.111111 | 5.4 |
| 215721_at | IGHG1 | | 0.510101 | 0.166667 | 66.9 | 0.060606 | 8.8 |
| 211798_x_at | IGLJ3 | 6 | 0.757576 | 0.30303 | 974.6 | 0.5 | 137.5 |
| 216846_at | IGLC2 /// | 6 | 0.700337 | 0.30303 | 32.2 | 0.166667 | 14.4 |
| | IGLV@ /// | | | | | | |
| | IGLJ3 | | | | | | |
| 211644_x_at | IGKC | 7 | 0.744108 | 0.272727 | 2760.2 | 0.277778 | 65.6 |
| 211881_x_at | IGLJ3 | 7 | 0.740741 | 0.272727 | 989.7 | 0.388889 | 159.3 |
| 217169_at | IGHG3 | 7 | 0.739057 | 0.272727 | 69.2 | 0.222222 | 14.3 |
| 216535_at | IGSF4B | 7 | 0.617845 | 0.272727 | 191.5 | 0.222222 | 46.1 |

### Cited Literature

(1) Goldhirsch A, Wood WC, Gelber RD, Coates AS, Thulimann B, Senn HJ. Meeting Highlights: updated international expert consensus on the primary therapy of early breast cancer. J Clin Oncol 21: 3357-3365, 2003
(2) Early Breast Cancer Trialists.' Collaborative Group. Polychemotherapy for early breast cancer: an overview of the randomised trials. Lancet 352: 930-942, 1998
(3) Early Breast Cancer Trialists' Collaborative Group. Tamoxifen for early breast cancer: an overview of the randomised trials. Lancet 351: 1451-1467, 1998
(4) Shapiro CL and Recht A. Side effects of adjuvant tratment of breast cancer. N Engl J Med 344: 1997-2008, 2001
(5) Altman DG and Lyman GH. Methodological challenges in the evaluation of prognostic factors in breast cancer. Br Cancer Res Treat 52: 289-303, 1998
(6) Jatoli I, Hilsenbeck SG, Clark GM, Osborne CK. Significance of axillary lymph node metastasis in primary breast cancer. J Clin Oncol 17: 2334-2340, 1999
(7) Van't Veer LJ, Dai H, van de Vijver MJ, He YD, Hart AAM, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature 415: 530-536, 2002
(8) Perou CM, Sorlie T, Eisen MB, van de Rijn M, Jeffrey SS, Rees CA, Pollack JR, Ross DT, Johnsen H, Akslen LA et al. Molecular portraits of human breast tumours. Nature 406: 747-752, 2000
(9) Slamon DJ, Clark GM, Wong SG, Levin WJ, Ullrich A, McGuire WL: Human breast cancer: correlation of relapse and survival with amplification of the ERBB2 oncogene. Science 1987, 235:177-181 1
(10) Reese D, Slamon DJ: ERBB2 signal transduction in human breast and ovarian cancer. Stem Cells 1997, 15:1-8
(11) Reese D, Slamon DJ: ERBB2 signal transduction in human breast and ovarian cancer. Stem Cells 1997, 15:1-8
(12) Salido M, Solé F, Tusquets I, Coromines JM, Espinet B, Marinoso ML, T Baró, MC Vela, X Fabregat, S Serrano, et al.: A comparative study of ERBB2 amplification and over expression using fluorescence in situ hybridization (FISH) and immunohistochemistry (IHC) in 101 breast cancer patients. Oncologia 2002, 4:255-259
(13) Bose S, Mohammed M, Shintaku P, Rao PN: ERBB2 gene amplification in low to moderately expressing breast cancers: possible role of chromosome 17/ERBB2 polysomy. Breast J 2001, 7:337-344
(14) An T, Sood U, Pietruk T, Cummings G, Hashimoto K, Crissman JD: In situ quantitation of inflammatory mononuclear cells in ducal infiltrating breast carcinoma. Am J Pathology 1987, 128:52-60
(15) Aaltomaa S, Lipponen P, Eskelinen M, Kosma VM, Marin S, Alhava E, Syrjänen K: Lymphocyte infiltrates as a prognostic variable in female breast cancer. Eur J Cancer 1992, 28:859-864
(16) Menard S, Tomasic G, Casalini P, Balsari A, Pilotti S, Cascinelli N, Salvadori B, Colnaghi MI, Rilke F: Lymphoid infiltration as a prognostic variable for early-onset breast carcinomas. Clin Can Res 1997, 3:817-819
(17) Lespagnard L, Gancberg D, Rouas G, Leclercq G, Somerhausen N De S, Di Leo A, Piccart M, Verhest A, Larsimont D: Tumour-infiltrationg dendritic cells in adenocarcinomas of the breast: A study of 143 neoplasms with a correlation to usual prognostic factors and to clinical outcome. Int J Cancer 1999, 84:309-314
(18) Disis et al: Existent T-cell and antibody immunity to HER-2/neu protein in patients with breast cancer. Cancer Res 1994, 54:16-20

## Claims

1. A method for the identification of a likely outcome of breast cancer in a breast cancer patient comprising the steps of
(a) determining the ERBB2 status of a tumour of said patient, and
(b) determining in a breast cancer tissue sample of said patient the expression level of at least one immunoglobulin in said tumour of said patient at least if said ERBB2 status of said tumour is positive,
wherein said identification of a likely outcome is based on said expression level if the ERBB2 status of said tumour is positive,
wherein the identification of the likely outcome comprises a classification of the patient into one of at least two groups, said two groups corresponding to a "good outcome" group and a "bad outcome" group, respectively,
and wherein a cancer is classified to be of the "bad outcome" group if the expression level of said at least one immunoglobulin is below a predetermined threshold level.

2. A method according to any of the preceding claims, wherein the step of determining the ERBB2 status of said tumour is prior to the step of determining the expression level of at least one immunoglobulin.

3. A method according to any of the preceding claims, wherein in step (b) the expression level of more than one immunoglobulin is determined.

4. A method according to any of the preceding claims, wherein the identification of the likely outcome is
(a) a determination of the likelihood of recurrence of a tumour in said patient, and/or
(b) the prediction of long term survival of said patient, and/or
(c) after surgery, and/or is
(d) the prognosis of breast cancer.

5. A method according to any of the preceding claims, wherein the determination of the ERBB2 status comprises a comparison of an ERBB2 expression level with a predetermined threshold value.

6. The method of claim 5, wherein said threshold value is determined from a Gaussian mixture model of a histogram determined from a suitable cohort of breast cancer patients, wherein the histogram comprises the ERBB2 expression levels in tumours of said cohort.

7. A method according to any of the preceding claims, wherein said determination of said expression level of at said least one immunoglobulin comprises the determination of the amount of mRNA coding for said immunoglobulin.

8. A method according to claim 7, wherein said determination of said expression level of at said least one immunoglobulin comprises the determination of the amount of mRNA coding for at least parts of said immunoglobulin.

9. A method according to any of the preceding claims, wherein the ERBB2 status is determined by PCR, or by real time PCR, or by immunohistochemistry, or by fluorescence in situ hybridisation (FISH), or by chromosome in situ hybridisation (CISH), or on a gene chip.

10. A method according to any of the preceding claims, wherein the expression level of said immunoglobulin is determined by PCR or real time PCR or by immunohistochemistry, or on a gene chip.

11. A method of any of the preceding claims, wherein the ERBB2 status and the expression level of said immunoglobulin are determined on the same gene chip.

12. A method according to any of the preceding claims, wherein said immunoglobulin is selected from the group consisting of IGH@, IGHG1, IGHG4, IGHM, IGHD, IGHG3, IGJ, IGKC,
IGLC2, IGLV@, IGLJ3, IGSF2, IGSF3, IGSF4B and IGSF4C.

13. A method according to claim 12, wherein the immunoglobulin is IGCK or IGHG1 or IGH@.

14. Use of a kit for performing the method according to any of claims 1-13, said kit comprising
(a) means for the determination of the ERBB2 status of a breast cancer patient from a tumour sample of said patient,
(b) means for the determination of the expression level of at least one immunoglobulin in a tumour sample of said patient, and
(c) printed material or a data carrier comprising instructions for performing a method according to any of claims 1-13.

15. Use of an apparatus for performing the method according to any of claims 1-13, said apparatus comprising
(a) means for the determination of the ERBB2 status of a breast cancer patient from a tumour sample of said patient,
(b) means for the determination of the expression level of at least one immunoglobulin in a breast cancer tissue sample of said patient, and
(c) computing means adapted for the identification of said likely outcome, wherein said identification is based on the expression level of said at least one immunoglobulin if said ERBB2 status is positive,
wherein the identification of the likely outcome comprises a classification of the patient into one of at least two groups, said two groups corresponding to a "good outcome" group and a "bad outcome" group, respectively,
and wherein a cancer is classified to be of the "bad outcome" group if the expression level of said at least one immunoglobulin is below a predetermined threshold level.

## Patentansprüche

1. Verfahren zur Identifikation des wahrscheinlichen Ausgangs von Brustkrebs bei einem Brustkrebspatienten, umfassend die folgenden Schritte:
(a) Bestimmen des ERBB2-Status eines Tumors des Patienten; und
(b) Bestimmen des Expressionsniveaus wenigstens eines Immunglobulins in einer Brustkrebsgewebeprobe des Patienten in dem Tumor des Patienten, wenigstens wenn der ERBB2-Status des Tumors positiv ist;
wobei die Identifikation des wahrscheinlichen Ausgangs auf dem Expressionsniveau beruht, wenn der ERBB2-Status des Tumors positiv ist;
wobei die Identifikation des wahrscheinlichen Ausgangs eine Klassifizierung des Patienten in eine von wenigstens zwei Gruppen umfasst, wobei die zwei Gruppen einer Gruppe mit "guter Prognose" bzw. einer Gruppe mit "schlechter Prognose" entsprechen und wobei ein Krebs als einer der Gruppe mit "schlechter Prognose" klassifiziert wird, wenn das Expressionsniveau des wenigstens einen Immunglobulins unter einem vorbestimmten Schwellenwert liegt.

2. Verfahren gemäß einem der vorstehenden Ansprüche, wobei der Schritt des Bestimmens des ERBB2-Status des Tumors vor dem Schritt des Bestimmens des Expressionsniveaus wenigstens eines Immunglobulins liegt.

3. Verfahren gemäß einem der vorstehenden Ansprüche, wobei in Schritt (b) das Expressionsniveau von mehr als einem Immunglobulin bestimmt wird.

4. Verfahren gemäß einem der vorstehenden Ansprüche, wobei es sich bei der Identifikation des wahrscheinlichen Ausgangs um
(a) eine Bestimmung der Wahrscheinlichkeit eines Rezidivs eines Tumors bei dem Patienten und/oder
(b) die Vorhersage der langfristigen Überlebenschancen des Patienten und/oder
(c) nach einer Operation und/oder
(d) die Prognose von Brustkrebs
handelt.

5. Verfahren gemäß einem der vorstehenden Ansprüche, wobei die Bestimmung des ERBB2-Status einen Vergleich eines ERBB2-Expressionsniveaus mit einem vorbestimmten Schwellenwert umfasst.

6. Verfahren gemäß Anspruch 5, wobei der Schwellenwert anhand eines Gaußschen Mischverteilungsmodells eines Histogramms bestimmt wird, das aus einer geeigneten Kohorte von Brustkrebspatienten bestimmt wurde, wobei das Histogramm die ERBB2-Expressionsniveaus in Tumoren der Kohorte umfasst.

7. Verfahren gemäß einem der vorstehenden Ansprüche, wobei die Bestimmung des Expressionsniveaus des wenigstens einen Immunglobulins die Bestimmung der Menge an mRNA, die für das Immunglobulin codiert, umfasst.

8. Verfahren gemäß Anspruch 7, wobei die Bestimmung des Expressionsniveaus des wenigstens einen Immunglobulins die Bestimmung der Menge an mRNA, die wenigstens für Teile des Immunglobulins codiert, umfasst.

9. Verfahren gemäß einem der vorstehenden Ansprüche, wobei der ERBB2-Status durch PCR oder durch Echtzeit-PCR oder durch Immunhistochemie oder durch Fluoreszenz-in-situ-Hybridisierung (FISH) oder durch Chromosomen-in-situ-Hybridisierung (CISH) oder auf einem Genchip bestimmt wird.

10. Verfahren gemäß einem der vorstehenden Ansprüche, wobei das Expressionsniveau des Immunglobulins durch PCR oder durch Echtzeit-PCR oder durch Immunhistochemie oder auf einem Genchip bestimmt wird.

11. Verfahren gemäß einem der vorstehenden Ansprüche, wobei der ERBB2-Status und das Expressionsniveau des Immunglobulins auf demselben Genchip bestimmt werden.

12. Verfahren gemäß einem der vorstehenden Ansprüche, wobei das Immunglobulin aus der Gruppe ausgewählt ist, die aus IGH@, IGHG1, IGHG4, IGHM, IGHD, IGHG3, IGJ, IGKC, IGLC2, IGLV@, IGU3, IGSF2, IGSF3, IGSF4B und IGSF4C besteht.

13. Verfahren gemäß Anspruch 12, wobei es sich bei dem Immunglobulin um IGCK oder IGHG1 oder IGH@ handelt.

14. Verwendung eines Kits zum Durchführen des Verfahrens gemäß einem der Ansprüche 1 bis 13, wobei der Kit Folgendes umfasst:
(a) Mittel zur Bestimmung des ERBB2-Status eines Brustkrebspatienten anhand einer Tumorprobe des Patienten;
(b) Mittel zur Bestimmung des Expressionsniveaus wenigstens eines Immunglobulins in einer Tumorprobe des Patienten; und
(c) gedrucktes Material oder einen Datenträger mit Anweisungen zur Durchführung eines Verfahrens gemäß einem der Ansprüche 1 bis 13.

15. Verwendung einer Vorrichtung zur Durchführung des Verfahrens gemäß einem der Ansprüche 1 bis 13, wobei die Vorrichtung Folgendes umfasst:
(a) Mittel zur Bestimmung des ERBB2-Status eines Brustkrebspatienten anhand einer Tumorprobe des Patienten;
(b) Mittel zur Bestimmung des Expressionsniveaus wenigstens eines Immunglobulins in einer Brustkrebsgewebeprobe des Patienten; und
(c) Recheneinrichtungen, die für die Identifikation des wahrscheinlichen Ausgangs geeignet sind, wobei die Identifikation auf dem Expressionsniveau des wenigstens einen Immunglobulins beruht, wenn der ERBB2-Status positiv ist;
wobei die Identifikation des wahrscheinlichen Ausgangs eine Klassifizierung des Patienten in eine von wenigstens zwei Gruppen umfasst, wobei die zwei Gruppen einer Gruppe mit "guter Prognose" bzw. einer Gruppe mit "schlechter Prognose" entsprechen und wobei ein Krebs als einer der Gruppe mit "schlechter Prognose" klassifiziert wird, wenn das Expressionsniveau des wenigstens einen Immunglobulins unter einem vorbestimmten Schwellenwert liegt.

## Revendications

1. Procédé d'identification d'une évolution possible du cancer du sein chez un patient atteint de cancer du sein comprenant les étapes consistant à
(a) déterminer le statut ERBB2 d'une tumeur dudit patient, et
(b) déterminer dans un échantillon de tissu de cancer du sein dudit patient le taux d'expression d'au moins une immunoglobuline dans ladite tumeur dudit patient au moins si ledit statut ERBB2 de ladite tumeur est positif,
dans lequel ladite identification d'une évolution possible est basée sur le taux d'expression si le statut ERBB2 de ladite tumeur est positif,
dans lequel l'identification de l'évolution possible comprend une classification du patient dans l'un d'au moins deux groupes, lesdits deux groupes correspondant à un groupe « évolution positive » et à un groupe « évolution négative », respectivement,
et où un cancer est classé dans le groupe « évolution négative » si le taux d'expression de ladite au moins une immunoglobuline est en-dessous d'un niveau de seuil prédéterminé.

2. Procédé selon l'une quelconque des revendications précédentes, dans lequel l'étape de détermination du statut ERBB2 de ladite tumeur est antérieure à l'étape de détermination du taux d'expression d'au moins une immunoglobuline.

3. Procédé selon l'une quelconque des revendications précédentes, dans lequel dans l'étape (b) le taux d'expression de plus d'une immunoglobuline est déterminé.

4. Procédé selon l'une quelconque des revendications précédentes, dans lequel l'identification de l'évolution possible est
(a) une détermination de la probabilité de récidive d'une tumeur chez ledit patient, et/ou
(b) la prédiction d'une survie à long terme dudit patient, et/ou
(c) après intervention chirurgicale, et/ou est
(d) le pronostic d'un cancer du sein.

5. Procédé selon l'une quelconque des revendications précédentes, dans lequel la détermination du statut ERBB2 comprend une comparaison d'un taux d'expression ERBB2 avec une valeur seuil prédéterminée.

6. Procédé selon la revendication 5, dans lequel ladite valeur de seuil est déterminée à partir d'un modèle de mélange gaussien d'un histogramme déterminé à partir d'une cohorte appropriée de patients atteints de cancer du sein, où l'histogramme comprend les niveaux d'expression ERBB2 dans les tumeurs de ladite cohorte.

7. Procédé selon l'une quelconque des revendications précédentes, dans lequel ladite détermination dudit taux d'expression de ladite au moins une immunoglobuline comprend la détermination de la quantité d'ARNm codant pour ladite immunoglobuline.

8. Procédé selon la revendication 7, dans lequel ladite détermination dudit taux d'expression de la au moins une immunoglobuline comprend la détermination de la quantité d'ARNm codant pour au moins des parties de ladite immunoglobuline.

9. Procédé selon l'une quelconque des revendications précédentes, dans lequel le statut ERBB2 est déterminé par PCR ou PCR en temps réel, ou par immunohistochimie, ou par hybridation in situ en fluorescence (FISH), ou par hybridation in situ chromosomique (CISH), ou sur une puce à ADN.

10. Procédé selon l'une quelconque des revendications précédentes, dans lequel le taux d'expression de ladite immunoglobuline est déterminé par PCR ou PCR en temps réel ou par immunohistochimie, ou sur une puce à ADN.

11. Procédé selon l'une quelconque des revendications précédentes, dans lequel le statut ERBB2 et le taux d'expression de ladite immunoglobuline sont déterminés sur la même puce à ADN.

12. Procédé selon l'une quelconque des revendications précédentes, dans lequel ladite immunoglobuline est sélectionnée parmi le groupe composé de IGH@, IGHG1, IGHG4, IGHM, IGHD, IGHG3, IGJ, IGKC, IGLC2, IGLV@, IGLJ3, IGSF2, IGSF3, IGSF4B et IGSF4C.

13. Procédé selon la revendication 12, dans lequel l'immunoglobuline est IGCK ou IGHG1 ou IGH@.

14. Utilisation d'un kit pour réaliser le procédé selon l'une quelconque des revendications 1 à 13, ledit kit comprenant :
(a) des moyens pour déterminer le statut ERBB2 d'un patient atteint de cancer du sein à partir d'un échantillon de tumeur dudit patient, et
(b) des moyens pour déterminer le taux d'expression d'au moins une immunoglobuline dans un échantillon de tumeur dudit patient, et
(c) des documents imprimés ou un support d'informations comprenant des instructions pour réaliser le procédé selon l'une quelconque des revendications 1 à 13.

15. Utilisation d'un appareil pour réaliser le procédé selon l'une quelconque des revendications 1 à 13, ledit appareil comprenant
(a) des moyens pour déterminer le statut ERBB2 d'un patient atteint de cancer du sein à partir d'un échantillon de tumeur dudit patient,
(b) des moyens pour déterminer le taux d'expression d'au moins une immunoglobuline dans un échantillon de tissu de cancer du sein dudit patient, et
(c) des moyens informatiques adaptés à l'identification de ladite évolution possible, dans lesquels ladite identification est fondée sur le taux d'expression de ladite au moins une immunoglobuline si ledit statut ERBB2 est positif,
dans lesquels l'identification de l'évolution possible comprend une classification du patient dans l'un d'au moins deux groupes, lesdits deux groupes correspondant à un groupe « évolution positive » et à un groupe « évolution négative », respectivement
et dans lesquels un cancer est classifié dans le groupe « évolution négative » si le taux d'expression de ladite au moins une immunoglobuline est inférieur à un niveau de seuil prédéterminé.
